# EUROPÄISCHE PATENTSCHRIFT

(11) **EP 3 503 938 B1**
(45) Veröffentlichungstag und Bekanntmachung des Hinweises auf die Patenterteilung: **10.03.2021**
(21) Anmeldenummer: 17767731.7
(22) Anmeldetag: 25.08.2017
(51) Int. Cl.: A61M 1/10

(54) **BLUTKREISLAUF-UNTERSTÜTZUNGSVORRICHTUNG**
CIRCULATORY ASSISTANCE DEVICE
DISPOSITIF D'ASSISTANCE DE LA CIRCULATION SANGUINE

(30) Priorität: 26.08.2016 DE 102016115940
(43) Veröffentlichungstag der Anmeldung: 03.07.2019
(73) Patentinhaber: Friedrich-Alexander-Universität Erlangen-Nürnberg, 91054 Erlangen (DE)
(72) Erfinder: ALKASSAR, Muhannad, 90425 Nürnberg (DE)
(74) Vertreter: Rösler Rasch van der Heide & Partner
(86) Internationale Anmeldenummer: PCT/EP2017/071422
(87) Internationale Veröffentlichungsnummer: WO 2018/037111

(56) Entgegenhaltungen:
- WO-A1-2004/075953
- US-A1- 2004 010 180

## Beschreibung

Die Erfindung betrifft eine Blutkreislauf-Unterstützungsvorrichtung für ein Herz eines Lebewesens.

Jährlich erkranken in Deutschland ca. 300 000 erwachsene Menschen neu an einer Herzinsuffizienz. Rund ein Drittel dieser Patienten leiden an einer derart schweren Herzinsuffizienz, dass sie innerhalb von zwei Jahren, mangels passender Behandlungsmöglichkeiten, versterben.

Im Kindesalter sind angeborene Herzfehler die häufigste Ursache für Herz-Kreislauf-Erkrankungen. Die Kriterien, die zur Beurteilung der Schwere des Defektes herangezogen werden, sind die Herzfunktion und strukturelle Anomalien. Die häufigsten strukturellen Defekte lassen sich operativ behandeln. Bei Beeinträchtigungen der Herzfunktion ist der Handlungsspielraum sehr eng. Lange Zeit gab es alternativ zu einer Herz-Transplantation keine Möglichkeit der adäquaten Behandlung bei einer lebensbedrohlichen Verschlechterung der Herzfunktion.

Im Verlauf der letzten Jahre wurden verschiedene Pumpsysteme entwickelt, die das insuffiziente Herz in seiner Funktion unterstützen. Hierbei handelt es sich immer um mechanische Pumpeinheiten, die parallel zum arbeitenden Herzen das Blut beschleunigen und somit das Herz indirekt entlasten.

Bisher war es dabei nur möglich, eine schwache Pumpleistung des Herzens durch eine direkte Beschleunigung des Blutes mit einer externen Pumpe zu kompensieren. Hierfür muss das Blut als Fördermedium immer mit Bestandteilen der Pumpe (Schlauchsystem, Pumpeinheit etc) in Kontakt treten. Dies führt infolge der starken Aktivierung des Gerinnungssystems immer zu einer starken Erhöhung des Risikos für die Bildung von Thromben. Des Weiteren erhöht die Implantation zu- und abführender Schlauchsysteme in die großen Gefäße die Wahrscheinlichkeit für plötzliche Blutungen. Ein solches System ist sehr anfällig und dient immer nur als Zwischenlösung bis zu einer Herztransplantation oder Regeneration des Herzens, so dass eine Entlassung des Patienten aus dem Krankenhaus unter dieser Therapie nicht möglich ist.

Aus der WO 2004/078025 A2 ist eine Blutkreislauf-Unterstützungsvorrichtung bekannt, bei der ein rohrförmiges Blutgefäß von einer Manschette umschlossen ist, die außenseitig mittels eines inkompressiblen Fluides druckbeaufschlagbar ist, wobei das Fluid mit einer separaten Pumpvorrichtung druckbeaufschlagt wird, in der ein Ballonvolumen mittels einer ringförmigen dielektrischen Elastomermembran periodisch radial komprimiert wird. Dadurch wird das Fluid pulsierend in die Manschette gedrückt und verengt damit das umschlossene Blutgefäß. Gesteuert wird die Pumpe von einem Sensor für den kardialen Zyklus des Herzens.

Diese Vorrichtung erfordert die Implantation zweier voluminöser Bauteile im menschlichen Körper, nämlich der Manschette und der Pumpe. Darüber hinaus bewirkt die periodische radiale Kompression eines Blutgefässes zunächst einen Blutstrom in beiden Axialrichtungen des Blutgefäßes, was einen Einsatz vornehmlich in Venen mit Venenklappen indiziert, jedoch auch dort den Wirkungsgrad der Pumpe verschlechtert.

Dielektrische Elastomere bestehen aus einer stark inkompressiblen elastisch verformbaren Elastomerfolie (beispielsweise aus Silicon, Naturkautschuk, Polyurethan oder Acryl), die beidseitig mit dehnbaren Elektroden beschichtet wird. Bei Spannungsbeaufschlagung der Elektroden verformt sich das Elastomer reversibel, wobei sich die Dicke der Elastomerfolie verringert und die Elastomerfolie sich gleichzeitig in beiden Richtungen senkrecht zur Dickenrichtung verlängert. Bei Unterbrechung der Stromversorgung formt sich das Elastomer wieder in den Ausgangszustand zurück. Bei einer ringförmigen dielektrischen Elastomermembran fällt eine dieser beiden Richtungen mit der Umfangsrichtung des Rings zusammen, was bedeutet, dass eine ringförmige dielektrische Elastomermembran unter Spannungseinwirkung einen vergrößerten Durchmesser aufweist.

Aus der US 2004/0249236 A1 ist eine ist eine Blutkreislauf-Unterstützungsvorrichtung gemäß dem Oberbegriff des Anspruchs 1 bekannt, die längliche dielektrische Elastomermembrane umfasst, die so befestigt sind, dass deren elektrische Anregung eine Kontraktion der das Herz umschließenden Manschette bewirkt. Nachteilig ist, dass die dielektrischen Elastomermembrane relativ kurz sind und damit nur geringe Kontraktionshübe möglich sind. Eine zweite Anordnung beansprucht viel Raum außerhalb des Herzens, was nachteilig ist.

Aus der US 2004/0010180 A1 ist eine Blutkreislauf-Unterstützungsvorrichtung gemäß dem Oberbegriff des Anspruchs 1 bekannt, die eine Membran aus einem strumpfartigen Geflecht dielektrischer Elastomerstränge umfasst. Unterhalb des Strumpfes kann ein fluidgefüllter Hohlraum zur Größenanpassung angeordnet sein. Bei einem Stromausfall kontraktiert die Manschette und verbliebt in diesem Zustand, was zu einer sofortigen massiven und lebensbedrohlichen Beeinträchtigung führen kann.

Die WO 2004/075953 A1 offenbart ebenfalls eine Blutkreislauf-Unterstützungsvorrichtung gemäß dem Oberbegriff des Anspruchs 1.

Es sind auch peristaltische Pumpen unter Verwendung von dielektrischen Elastomermembranen bekannt, die außerhalb des menschlichen oder tierischen Körpers angeordnet sind.

Aus der US 8 100 819 B2 ist die Verwendung einer dielektrischen Elastomermembran als künstlicher ringförmiger Schließmuskel für Blase, Darm oder Speiseröhre sowie als am Herz fixierbarer Patch bekannt.

In der US 6 293 906 ist eine netzförmige Umhüllung für ein krankhaft vergrößertes Herz offenbart.

Die Aufgabe der Erfindung ist es, eine baulich einfache und mit hohem Wirkungsgrad arbeitende Blutkreislauf-Unterstützungsvorrichtung bereitzustellen.

Die Erfindung ergibt sich aus den Merkmalen des Anspruchs 1. Vorteilhafte Weiterbildungen und Ausgestaltungen sind Gegenstand der abhängigen Ansprüche. Die Aufgabe wird gemäß Patentanspruch 1 dadurch gelöst, dass eine Blutkreislauf-Unterstützungsvorrichtung für ein Herz eines Lebewesens bereitgestellt wird, umfassend eine Manschette zur periodischen Druckbeaufschlagung des Herzens mittels mindestens einer dielektrischen Elastomermembran, die von einer Steuereinrichtung in Synchronisation mit einem Herzschlag zur Blutförderung im Hohlraum pulsierend ansteuerbar ist, wobei die Manschette ausgebildet ist, außenseitig über das Herz gestülpt zu werden und dazu eine Innenform aufweist, die an die Außenkontur des Herzens wenigstens im Bereich außerhalb von Herzventrikeln angepasst ist, wobei die Manschette aus einer äußeren die dielektrische Elastomermembran umfassene Kontraktionsschicht sowie einer inneren Kissenschicht besteht und die Kissenschicht mit einer inkompressiblen Flüssigkeit gefüllt ist und mindestens ein Austrittsventil aufweist, das in einem Normalzustand geschlossen und in einem Notzustand geöffnet ist.

Die Erfindung stellt ein mechanisches System bereit, das unter Ausnutzung der strukturellen Gegebenheiten eines kranken Herzens ganz spezifisch das Herz in seiner Pumpfunktion unterstützt. Dieses weist im Vergleich zu herkömmlichen Herz unterstützenden Pumpsystemen viele Vorteile auf, die eine dauerhafte Unterstützung kranker Herzen ermöglicht. Eine Unterstützung der eigenen Pumpfunktion des Herzens durch die von außen an das Herz angelegte Manschette birgt keine erhöhte Gefahr für eine Blutungs- und Thromboseneigung. Durch das Fehlen einer zusätzlichen mechanischen Pumpe muss keine aufwendige äußere Apparatur an den Patienten angeschlossen werden. Dies führt zu einer deutlichen Steigerung der Lebensqualität des herzkranken Patienten. Durch die Kissenschicht wird vorteilhafterweise erreicht, dass die Manschette bei einem Energieausfall, welcher einen Notzustand darstellt, bei dem die dielektrische Elastomermembran, die ohne elektrische Energiezufuhr den Zustand mit geringerer Ausdehnung, also mit geringerem Umfang, einnimmt, nicht das Herz einschnürt, sondern vielmehr die in der zwischen Kontraktionsschicht und Herzwand angeordneten Kissenschicht befindliche inkompressible Flüssigkeit durch die automatische Öffnung des Austrittsventils aus der Kissenschicht austreten kann. Damit lässt sich diese Auswirkung der verengten Kontraktionsschicht teilweise oder ganz vermeiden. In die Kissenschicht ist vorzugsweise im Bereich der Verbindungsstelle mit dem Herzen ein Membranventilsystem integriert, welches bei Stromausfall öffnet und zu einem Austritt der inkompressiblen Flüssigkeit führt. Bei geschlossenem Austrittsventil ist die Kissenschicht verformbar und folgt den Formänderungen der Elastomermembran.

Ein Einsatz des erfindungsgemäßen Herzunterstützungssystems über längere Zeit ist möglich. Durch die unmittelbare Einwirkung auf das Herz ist auch ein höherer Wirkungsgrad erzielbar, so dass der Energiebedarf pro Zeiteinheit gegenüber herkömmlichen Systemen geringer ist und die erfindungsgemäße Blutkreislauf-Unterstützungsvorrichtung damit bei einem begrenzt verfügbaren Energievorrat länger funktionsfähig ist. Sofern also eine Energieversorgungseinrichtung im Körper untergebracht wird, ist der Patient damit länger unabhängig von einem Aufladevorgang, was die Lebensqualität verbessert.

Unter einer an das Herz anlegbaren Manschette wird gemäß einer Weiterbildung eine im Bereich der Ventrikel angelegte Manschette verstanden.

Gemäß einer alternativen Weiterbildung wird unter einer an das Herz anlegbaren Manschette eine im Bereich der Ventrikel und wenigstens teilweise auch im Bereich mindestens eines Atriums angelegte Manschette verstanden, wobei die Manschette mindestens zwei unabhängig voneinander arbeitende dielektrische Elastomermembranen enthalten muss, da eine Druckbeauschlagung der Atrien zu einem anderen Zeitpunkt im kardialen Zyklus erfolgen muss als die Druckbeauschlagung der Ventrikel.

In einer besonderen Weiterbildung wird eine dielektrische Elastomermembran bereitgestellt, die aus mindestens zwei Teilmembranen besteht, welche von der Steuereinrichtung unterschiedlich angesteuert werden. Dabei ist eine erste Teilmembran im Bereich der Atrien (Herz-Vorhöfe) angeordnet und eine zweite Teilmembran ist im Bereich der Ventrikel (Herz-Kammern) angeordnet. Die unterschiedliche Ansteuerung erfolgt derart, dass in Abstimmung mit der Sinusknoten- und/oder Atrioventrikularknoten-Erregung die zwei Teilmembranen antizyklisch angesteuert werden. Die erste und zweite Teilmembran können in Form von zwei voneinander getrennten Elastomermembranen realisiert sein, die jeweils über getrennte Elektroden von der Steuereinrichtung angesteuert werden. Alternativ sind die erste und zweite Teilmembran über eine gemeinsame Elastomermembran realisiert, wobei die Elektrodenschichten entsprechend unterbrochen und mit eigenen Stromversorgungsanschlüssen versehen sind, um die Teilmembranen auszubilden. Statt zwei können auch drei oder vier Teilmembranen vorgesehen sein.

Gemäß einer vorteilhaften Weiterbildung dieser Ausbildung weist die Kissenschicht eine Dicke auf, die mindestens einem Kontraktionshub der Kontraktionsschicht entspricht. Auf diese Weise wird eine Beeinträchtigung der Herztätigkeit durch Stromausfall vollständig vermieden, weil die Kontraktionsschicht im energielosen Zustand nicht enger am Herzen anliegt als im funktionsfähigen Zustand, da die sich entleerende Kissenschicht damit einen kompressiblen Puffer bildet.

Vorzugsweise ist die Manschette tulpenartig ausgebildet zur vollständigen Umschließung der Herzspitze. Dadurch wird eine größere Einwirkung auf das Herz erzielt als bei einer nur ringförmigen Umschließung. Gemäß einer tulpenartigen Ausbildung verengt sich der für die Umschließung der Herzspitze vorgesehene Bereich der Manschette in Richtung der Herzspitze oder schließt in einer geschlossenen Spitze ab, wohingegen sich die Manschette in Richtung der Herzvorhöfe, in deren Bereich auch das Herz einen größeren Umfang aufweist, erweitert.

Gemäß einer vorteilhaften Weiterbildung der Erfindung besteht die Kontraktionsschicht aus einer Anzahl Ringabschnitte, die separat sequenziell zeitversetzt elektrisch ansteuerbar sind. Damit ist es möglich, synchron mit der von der Herzspitze ausgehenden Kontraktion des Herzmuskels eine unterstützende Kontraktion der Membran zu bewirken. Vorzugsweise sind dazu 3 bis 250, besonders bevorzugt 5 bis 20 Ringsabschnitte der Kontraktionsschicht vorgesehen. Die Ansteuerung der einzelnen Ringsabschnitte erfolgt zeitversetzt gemäß der vorher bestimmten typischen Ausbreitung der muskulären Kontraktionsbewegung.

Gemäß einer vorteilhaften Weiterbildung dieser Ausbildung besteht die Kissenschicht aus der gleichen Anzahl an fluidgefüllten Ringräumen, die jeweils unterhalb der Kontraktionsschicht-Ringabschnitte liegen, wobei jeder Ringraum ein eigenes Austrittsventil aufweist. Die benachbarten Ringräume sind vorzugsweise durch dehnbare Trennwände voneinander getrennt.

Gemäß einer vorteilhaften Weiterbildung der Erfindung kommuniziert die Kissenschicht über das Austrittsventil mit einem Auffangbeutel oder einer Austrittsleitung zur Ausleitung aus dem Körper. Damit wird vermieden, dass die in der Kissenschicht befindliche Flüssigkeit in die Körperhöhle austritt. Sofern die Flüssigkeit jedoch geeignet gewählt wird für den Austritt in die Brusthöhle, kann auf solche Bestandteile verzichtet werden. Nicht beschränkende Beispiele für geeignete Flüssigkeiten sind alle physiologisch verträglichen Flüssigkeiten, wie beispielsweise physiologische Kochsalzlösung, Plasmaexpander, etwa solche auf der Grundlage von Dextran, Hydroxyethylstärke oder Gelatine, oder weitere fachbekannte Flüssigkeiten. Die physiologisch verträgliche Flüssigkeit selbst ist vorzugsweise pharmakologisch inaktiv.

Gemäß einer vorteilhaften alternativen Weiterbildung der Erfindung ist daher vorgesehen, dass die inkompressible Flüssigkeit der Kissenschicht physiologisch verträglich ist und über das Austrittsventil in den Körper des Lebewesens, insbesondere in dessen Brusthöhle freisetzbar ist. Vorteilhaft braucht dadurch keine Austrittsleitung aus dem Körper bereitgestellt zu werden, die prinzipiell auch immer eine Eintrittspforte für Keime, Substanzen oder Verunreinigungen darstellen könnte.

Gemäß einer ganz besonderen Weiterbildung umfasst die inkompressible und physiologisch verträgliche Flüssigkeit der Kissenschicht wenigstens eine positiv inotrop wirkende Substanz. Nach Öffnung des Austrittsventils und der dadurch bedingten Freisetzung der Flüssigkeit in den Körper des Lebewesens, in der Regel also in dessen Brusthöhle, wird durch die wenigstens eine positiv inotrop wirkende Substanz eine wenigstens kurzzeitige Leistungssteigerung des Herzens bewirkt. Dadurch wird vorteilhaft einem Blutdruckabfall entgegengewirkt, der gerade in den ersten Augenblicken nach Ausfall der Blutkreislauf-Unterstützungsvorrichtung zu einer bedrohlichen Kreislaufschwäche führen könnte. Weiterhin wird dem Körper damit ein Signal gegeben, sich darauf einzustellen, dass die Pumpleistung nun wieder vollständig vom körpereigenen Herzen zu erbringen ist, bis der Ausfall der Blutkreislauf-Unterstützungsvorrichtung anderweitig medikamentös, chirurgisch oder durch medizinische Apparate kompensiert wird. Nicht beschränkende Beispiele für positiv inotrop wirkende Substanzen sind Adrenalin, Noradrenalin, Herzglykoside wie beispielsweise Digoxin, Digitoxin oder Quabain, Wirkstoffe aus der Gruppe der sogenannten Kalzium-Sensitizer wie etwa Levosimendan, Wirkstoffe aus der Gruppe der Phosphordiesterase-3-Hemmer, wie beispielsweise 3-Amino-5-(4-pyridinyl)-2(1*H*)-pyridinon (Amrinon), 6-[4-(1-Cyclohexyl-1*H*-tetrazol-5-yl)butoxy]-3,4-dihydrochinolin-2-on (Cilostazol), Milrinon oder Enoximon. Die physiologisch verträgliche Flüssigkeit kann auch Kombinationen von zweier oder mehrerer positiv inotrop wirkende Substanzen umfassen. Sofern keine Standardmenge an positiv inotrop wirkender Substanz oder Substanzen in der psychologisch verträglichen Flüssigkeit enthalten ist, kann der Fachmann die Wahl und die Dosierung der positiv inotrop wirkenden Substanz oder Substanzen erforderlichenfalls in Abhängigkeit vom Alter, dem Gewicht, der übrigen Medikamentierung, dem Krankheitszustand des Lebewesens und gegebenenfalls anderer Parameter geeignet bestimmen.

Gemäß einer vorteilhaften Weiterbildung der Erfindung umfasst die Steuereinrichtung eine Notzustand-Feststellungseinheit, die den Notzustand feststellt, wenn die Energiezufuhr für die Kontraktionsschicht für einen vorgegebenen oder vorgebbaren Zeitraum einen Mindestwert unterschreitet. Hiermit wird sichergestellt, dass ein Energieausfall zuverlässig festgestellt wird, um zu gewährleisten, dass einerseits ein signifikanter Energieausfall zuverlässig detektiert wird, andererseits jedoch auch kein Fehlalarm ausgelöst wird, der eine irreversible Öffnung des Austrittsventils auslösen und damit die erfindungsgemäße Blutkreislauf-Unterstützungsvorrichtung funktionell ausschalten würde. Sollte über einen von einem Kontrollgerät geregelten Zeitraum kein oder ein unzureichender Strom an das Ventil abgegeben werden, so führt dies über das geöffnete Ventil zu einem Verlust der Flüssigkeit zwischen Herz und Membran, so dass die nun in Kontraktion befindliche Membran das Herz nicht in seiner restlichen Eigenfunktion behindern kann.

Die Aufgabe wird ferner gelöst durch ein Blutkreislauf-Unterstützungssystem umfassend eine Kontrolleinrichtung, eine Blutkreislauf-Unterstützungsvorrichtung nach einem oder mehreren der vorherigen Ausführungsformen oder Weiterbildungen, eine Energieversorgungseinrichtung und mindestens eine Sensoreinrichtung zur Erfassung des kardialen Zyklus.

Die Erfindung betrifft ferner ein nicht beanspruchtes medizinisches Verfahren der Einführung eines oben erläuterten Blutkreislauf-Unterstützungssystems in den Körper eines Lebewesens, wobei die Blutkreislauf-Unterstützungsvorrichtung über das Herz des Lebewesens gestülpt und dort fixiert wird und die Sensoreinrichtung am Herzen fixiert wird.

Die Erfindung betrifft ferner ein nicht beanspruchtes Verfahren zur Unterstützung des Blutkreislaufs eines Lebewesens unter Verwendung des vorgenannten Blutkreislauf-Unterstützungssystems, wobei der kardiale Zyklus des Herzens erfasst wird und synchron dazu die Kontraktionsschicht mit Energie versorgt wird, so dass die Kontraktion der Elastomerschicht mit der Kontraktion des unter der Elastomerschicht liegenden Bereichs des Herzmuskels zusammenwirkt. Auf diese Weise lässt sich bei einem Patienten mit Herzinsuffizienz die Pumpleistung des Herzens verbessern und damit die Lebenserwartung und die Lebensqualität steigern.

Weitere Vorteile, Merkmale und Einzelheiten ergeben sich aus der nachfolgenden Beschreibung, in der - gegebenenfalls unter Bezug auf die Zeichnung - zumindest ein Ausführungsbeispiel im Einzelnen beschrieben ist. Gleiche, ähnliche und/oder funktionsgleiche Teile sind mit gleichen Bezugszeichen versehen.

Es zeigen:
- Figur 1: eine schematische Darstellung eines Herzens mit einem Blutkreislauf-Unterstützungssystem im normalen Betriebszustand;
- Figur 2:: eine schematische Darstellung eines Herzens mit dem Blutkreislauf-Unterstützungssystem in einem Notfallzustand;
- Figur 3:: eine schematische Darstellung eines Herzens mit einer zweiten Ausführung eines Blutkreislauf-Unterstützungssystems im normalen Betriebszustand,
- Figur 4:: eine schematische Darstellung eines Herzens mit einer dritten Ausführung eines Blutkreislauf-Unterstützungssystems,
- Figur 5:: mehrere Darstellungen der Wirkungsweise der dritten Ausführungsform.

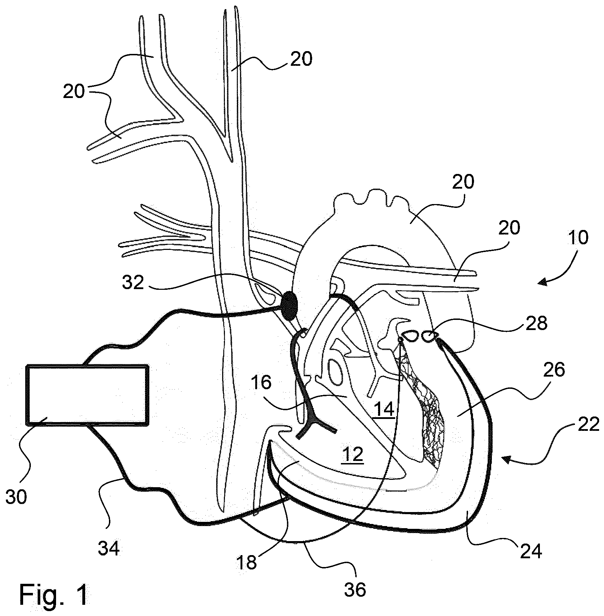

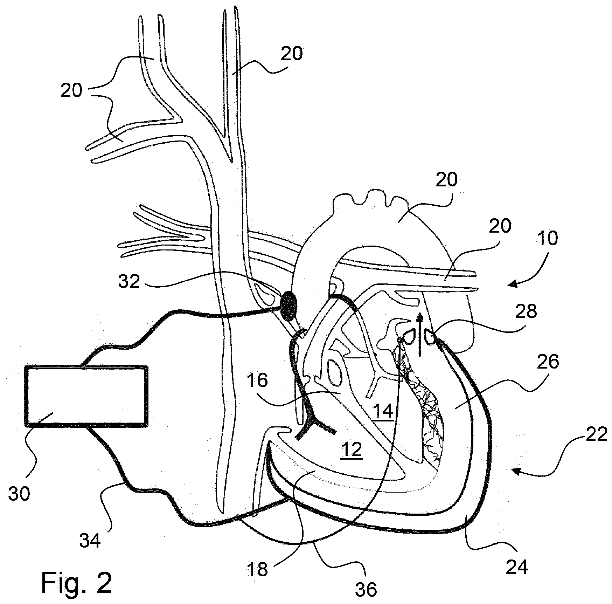

In den Figuren 1 und 2 ist ein Herz eines Lebewesens mit einem Blutkreislauf-Unterstützungssystem nach der vorliegenden Erfindung in zwei Zuständen dargestellt, nämlich in Figur 1 im normalen Betriebszustand und in Figur 2 in einem Notfallzustand. Das Herz 10 besteht im Wesentlichen aus einem rechten Ventrikel 12, einem linken Ventrikel 14, einer Ventrikelscheidewand 16 und einer Ventrikelwand 18. Weitere Gefäße des Blutkreislaufsystems 20 sind nicht näher dargestellt bzw. mit Bezugszeichen versehen.

Eine Manschette 22 einer Blutkreislauf-Unterstützungsvorrichtung umschließt in einer ersten Ausführungsform das Herz 10 passgenau während der Diastolenphase des kardialen Zyklus. Die Manschette umfasst eine äußere Kontraktionsschicht 24 und eine innere Kissenschicht 26. Im Inneren der Kontraktionsschicht 24 befindet sich mindestens eine elastische Elastomermembran, die vorzugsweise einen geschlossenen Ring oder, wie in Figur 1 und 2 dargestellt, eine Tulpenform aufweist. In der Manschette 22 können mehrere dielektrische Elastomermembranen neben- oder übereinander angeordnet sein. Die Elastomermembranen sind vorzugsweise geschlossen, jedoch ist es im Rahmen der Erfindung auch möglich, mehrere ringförmige getrennt angesteuerte Elastomermembranen nebeneinander anzuordnen.

Als Material für die Kontraktionsschicht 24 kommen beispielsweise PDMS, Polyurethan, Acrylate (zB VHB von 3M) in Betracht. Besonders geeignet ist Silicon mit Polydimethyl Siloxan als Polymerkomponente und Acrylpolymere und Naturkautschuk.

Die Kissenschicht 26 steht mit einem Austrittsventil 28 in Wirkverbindung derart, dass in der Kissenschicht 26 befindliche inkompressible Flüssigkeit im geöffneten Zustand des Austrittsventils 28 in die Umgebung oder in dafür vorgesehene, nicht dargestellte Reservoirs austreten kann. In Figur 1 befindet sich die Blutkreislauf-Unterstützungsvorrichtung im normalen Betriebszustand, sodass das Austrittsventil 28 geschlossen ist.

Die Kissenschicht 26 hat vorzugsweise eine Dicke von 0,5 bis 2,5 cm und ist vorzugsweise mit einer resorbierbaren wässrigen Lösung gefüllt. Gemäß einer Weiterbildung kann diese auch Medikamente enthalten.

Die Blutkreislauf-Unterstützungsvorrichtung 22 ist mit einer Kontrolleinrichtung 30 verbunden, die eine nicht gezeigte Energieversorgungseinheit umfasst. Die Kontrolleinrichtung 30 ist ferner mit einem Sensor 32 verbunden, der an geeigneter Stelle den kardialen Zyklus des Herzens 10 erfasst. Die Kontrolleinrichtung 30 ist über eine Stromversorgungsleitung 34 mit der Kontraktionsschicht 24 verbunden, wobei über separate Adern der Stromversorgungsleitung 34 auch Sensorsignale nicht dargestellter Sensoren über den Zustand der Kontraktionsschicht 24 laufen können. Von der Stromversorgungsleitung 34 zweigt eine Stromversorgungsleitung 36 ab, die das Austrittsventil 28 mit Energie versorgt und zwar derart, dass dieses bei Energieversorgung den in Figur 1 gezeigten geschlossenen Zustand einnimmt. Bei Ausfall der Energieversorgungseinheit der Kontrolleinrichtung 30 bzw. unzureichender Stromversorgung der Kontraktionsschicht 24 erhält das Austrittsventil 28 keine oder eine unzureichende Energieversorgung, wobei sich dieses öffnet, so dass das in der Kissenschicht 26 befindliche inkompressible Fluid über das Austrittsventil 28 in die Umgebung, d.h. das umgebende Gewebe im Brustraum, austreten kann, wie in Figur 2 dargestellt ist.

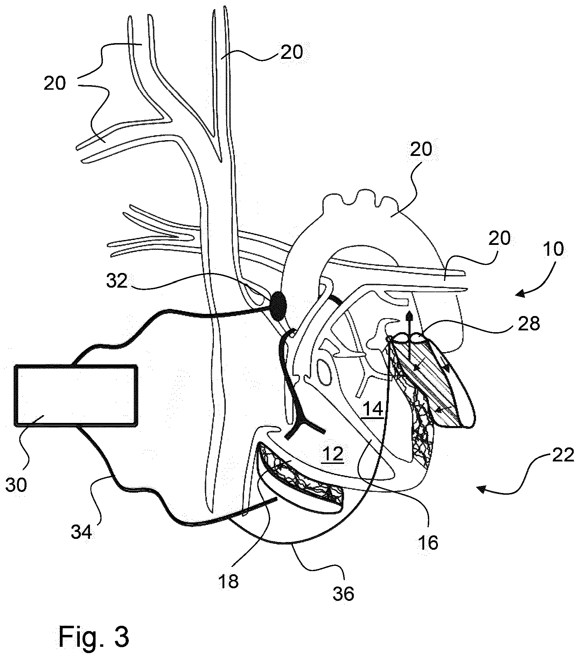

In Figur 3 ist eine zweite Ausführungsform eines Blutkreislauf-Unterstützungssystems in Form einer ringförmigen Manschette 40 dargestellt. Abgesehen davon, dass diese Manschette 40 die Spitze des Herzens 10 nicht umschließt, ist diese strukturell und wirkungstechnisch gleich aufgebaut wie die Manschette 22.

Wie in den Figuren 1 bis 3 zu sehen ist, erstreckt sich die Manschette 22 bzw. 40 vorzugsweise entlang der gesamten Ventrikelwand 18 und ist über nicht dargestellte Mittel daran befestigt, vorzugsweise ringförmig angelegt.

Im normalen Betrieb erfasst der Sensor 32 den kardialen Zyklus des Herzens 10. In einem Ausgangszustand befindet sich das Herz 10 in der diastolischen Phase, in der dieses entspannt ist und das größte Volumen einnimmt, wobei sich die beiden Ventrikel mit Blut füllen. In diesem Zustand wird die Kontraktionsschicht 24 mit Energie versorgt, sodass diese sich in einem Zustand größtmöglicher Ausdehnung befindet. Die mit einem inkompressiblen Fluid gefüllte Kissenschicht 26 liegt dabei innenseitig an der Herzwand und außenseitig an der Kontraktionsschicht 24 an. Sofern die Kontrolleinrichtung 30 den Beginn der Systole erfasst, wird die Energieversorgung von der Kontraktionsschicht 24 schlagartig oder nach einem vorgegebenen Ablauf unterbrochen, sodass sich die Kontraktionsschicht 24 damit radial nach innen zusammenzieht und über die inkompressible Kissenschicht 26 die dadurch entstehenden, nach radial innen gerichteten Kräfte auf die Ventrikelwand 18 des Herzens 10 überträgt. Spätestens wenn die systolische Phase des kardialen Zyklus abgeschlossen ist, wird die Kontraktionsschicht 24 wieder mit Energie versorgt, sodass diese sich wieder radial ausdehnt und mit ihr die Kissenschicht 26 von der sich entspannenden Ventrikelwand 18 wegbewegt.

Sowohl die Energiezufuhr als auch die Energieabschaltung über die Leitung 34 kann vorzugsweise nach einem vorgegebenen Spannungs-Zeitverlauf erfolgen.

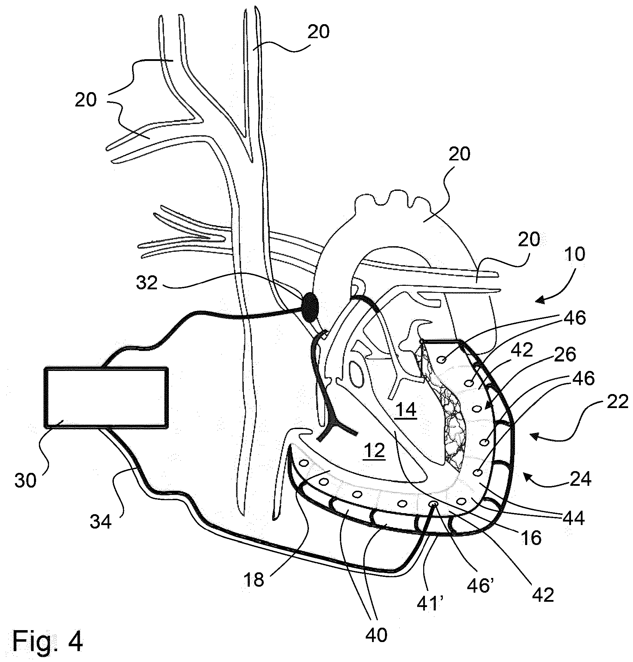

In Figur 4 ist eine dritte Ausführungsform dargestellt, die sich von den vorgenannten Ausführungen dadurch unterscheidet, dass die Kontraktionsschicht 24 aus einer Anzahl neben einander angeordneten Ringabschnitten 41, die alle einzeln von der Kontrolleinrichtung 30 angesteuert werden, was aus Gründen der Übersichtlichkeit nur für die einen mit dem Bezugszeichen 41' gekennzeichneten Ringabschnitt dargestellt ist. Es handelt sich normalerweise um eine Folie, die ringförmig untergliedert ist. Alternativ können auch einzelne miteinander verbundene Folienringe vorgesehen werden.

Entsprechend ist die darunter angeordnete Kissenschicht 26 in eine Anzahl nebeneinander liegender und durch dehnbare Trennwände 42 getrennte Ringräume 44 unterteilt. Jeder Ringraum 44 hat ein eigenes von der Kontrolleinrichtung gesteuertes Austrittsventil 46 (aus Gründen der Übersichtlichkeit nur für ein Austrittsventil 46' dargestellt) in die Umgebung.

Die Ringabschnitte 41 werden von der Kontrolleinrichtung 30 separat sequenziell zeitversetzt elektrisch angesteuert und zwar synchron mit der von der Herzspitze ausgehenden Kontraktion des Herzmuskels. Ansteuerung der einzelnen Ringsabschnitte 41 erfolgt also zeitversetzt gemäß der typischen Ausbreitung der muskulären Kontraktionsbewegung des Herzens.

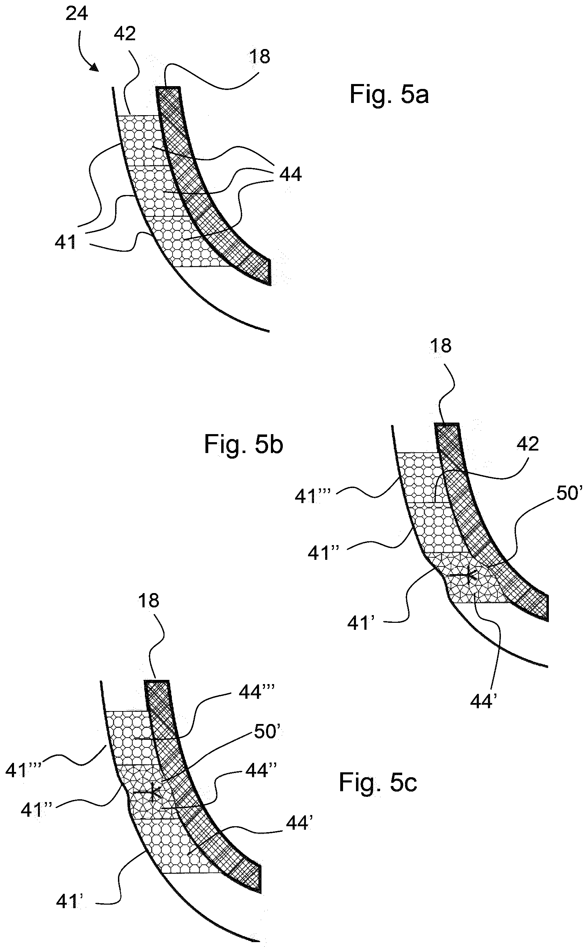

Figur 5 zeigt drei schematische Darstellungen zur Demonstration der Funktionsweise der Ausführung gemäß Figur 4. In Figur 5a ist die Kontraktionsschicht 24 in einer Ruhestellung gezeigt, bei der alle Ringabschnitte 41 mit Energie versorgt sind. Dementsprechend wird kein Druck auf die Ventrikelwand 18 ausgeübt.

In der Stellung gemäß Figur 5b ist die Energiezufuhr zum ersten Ringabschnitt 41' unterbrochen, nicht jedoch zu den weiteren Ringabschnitten 41" und 41'''. Somit zieht sich der erste Ringabschnitt 41' zusammen und verengt sich dabei. Da im zugehörigen Ringraum 44' inkompressibles Medium enthalten ist, wird damit ein Druck auf die Ventrikelwand 18 an der Stelle 50' ausgeübt.

Danach wird die Energiezufuhr zum ersten Ringabschnitt 41' wieder hergestellt und dafür die Energiezufuhr zum benachbarten zweiten Ringabschnitt 41" unterbrochen, so dass sich der erste Ringabschnitt 41' wieder ausdehnt und den Druck auf Die Stelle 50' der Ventrikelwand 18 nachlässt, während sich der zweite Ringabschnitt 42" zusammenzieht und an der Stelle 50" Druck auf die Ventrikelwand 18 erzeugt. Alles wird durch die Kontrolleinrichtung 30 gesteuert und zwar synchron zur Ausbreitung der Herzkontraktion von der Herzspitze aus. Auf diese Weise wird ein Ringabschnitt 41 nach dem anderen geschaltet und bewirkt damit eine Druckwelle, die sich ebenfalls synchron zur Ausbreitung der Herzkontraktion ausbreitet.

Obwohl die Erfindung im Detail durch bevorzugte Ausführungsbeispiele näher illustriert und erläutert wurde, so ist die Erfindung nicht durch die offenbarten Beispiele eingeschränkt. Vielmehr versetzen die vorhergehende Beschreibung und die Figurenbeschreibung den Fachmann in die Lage, die beispielhaften Ausführungsformen konkret umzusetzen, wobei der Fachmann in Kenntnis des offenbarten Erfindungsgedankens vielfältige Änderungen beispielsweise hinsichtlich der Funktion oder der Anordnung einzelner, in einer beispielhaften Ausführungsform genannter Elemente vornehmen kann, ohne den Schutzbereich zu verlassen, der durch die Ansprüche und deren rechtliche Entsprechungen, wie etwa weitergehenden Erläuterung in der Beschreibung, definiert wird.

## Patentansprüche

1. Blutkreislauf-Unterstützungsvorrichtung (22) für ein Herz eines Lebewesens, umfassend eine Manschette zur periodischen Druckbeaufschlagung des Herzens mittels mindestens einer dielektrischen Elastomermembran, die von einer Steuereinrichtung in Synchronisation mit einem Herzschlag zur Blutförderung pulsierend ansteuerbar ist, wobei die Manschette ausgebildet ist, außenseitig über das Herz (10) gestülpt zu werden und dazu eine Innenform aufweist, die an die Außenkontur des Herzens wenigstens im Bereich außerhalb von Herzventrikeln angepasst ist **dadurch gekennzeichnet, dass** die Manschette aus einer äußeren die dielektrische Elastomermembran umfassene Kontraktionsschicht (24) sowie einer inneren Kissenschicht (26) besteht und die Kissenschicht (26) mit einer inkompressiblen Flüssigkeit gefüllt ist und mindestens ein Austrittsventil (28) aufweist, das in einem Normalzustand geschlossen und in einem Notzustand geöffnet ist.

2. Blutkreislauf-Unterstützungsvorrichtung (22) nach Anspruch 1, **dadurch gekennzeichnet, dass** die Kissenschicht (26) eine Dicke aufweist, die mindestens einem Kontraktionshub der Kontraktionsschicht (24) entspricht.

3. Blutkreislauf-Unterstützungsvorrichtung (22) nach Anspruch 1, **dadurch gekennzeichnet, dass** die Kontraktionsschicht (24) aus einer Anzahl Ringabschnitte besteht, die separat sequenziell zeitversetzt elektrisch ansteuerbar sind.

4. Blutkreislauf-Unterstützungsvorrichtung (22) nach Anspruch 3, **dadurch gekennzeichnet, dass** 3 bis 250 Ringsabschnitte der Kontraktionsschicht (24) vorgesehen sind.

5. Blutkreislauf-Unterstützungsvorrichtung (22) nach Anspruch 3, **dadurch gekennzeichnet, dass** die Kissenschicht (26) aus der gleichen Anzahl an fluidgefüllten Ringräumen besteht, die jeweils unterhalb der Kontraktionsschicht-Ringabschnitte liegen, wobei jeder Ringraum ein eigenes Austrittsventil aufweist.

6. Blutkreislauf-Unterstützungsvorrichtung (22) nach einem der vorherigen Ansprüche, **dadurch gekennzeichnet, dass** die Kissenschicht (26) über das Austrittsventil (28) mit einem Auffangbeutel oder einer Austrittsleitung zur Ausleitung aus dem Körper kommuniziert.

7. Blutkreislauf-Unterstützungsvorrichtung (22) nach einem der vorherigen Ansprüche, **dadurch gekennzeichnet, dass** die inkompressible Flüssigkeit der Kissenschicht (26) physiologisch verträglich ist und über das Austrittsventil (28) in den Körper des Lebewesens freisetzbar ist.

8. Blutkreislauf-Unterstützungsvorrichtung (22) nach Anspruch 6, **dadurch gekennzeichnet, dass** die inkompressible Flüssigkeit der Kissenschicht (26) wenigstens eine positiv inotrop wirkende Substanz umfasst.

9. Blutkreislauf-Unterstützungsvorrichtung (22) nach einem der vorherigen Ansprüche, **dadurch gekennzeichnet, dass** die Steuereinrichtung eine Notzustand-Feststellungseinheit umfasst, die einen Notzustand feststellt, wenn die Energiezufuhr für die Kontraktionsschicht (24) für einen vorgegebenen Zeitraum einen Mindestwert unterschreitet.

10. Blutkreislauf-Unterstützungssystem umfassend eine Blutkreislauf-Unterstützungsvorrichtung (22) nach einem oder mehreren der vorherigen Ansprüche, eine Kontrolleinrichtung (30), eine Energieversorgungseinrichtung und mindestens eine Sensoreinrichtung zur Erfassung des kardialen Zyklus.

## Claims

1. A blood circulation assistance device (22) for a heart of a living being, comprising a cuff for periodically applying pressure to the heart by means of at least one dielectric elastomer membrane, which can be controlled by a control device in synchronization with a heartbeat in order to convey blood in pulses, wherein the cuff is configured to be pulled over the outside of the heart (10) and to this end has an internal shape which is adapted to the external contour of the heart at least in the region outside the ventricles of the heart, **characterized in that** the cuff is composed of an outer contraction layer (24) comprising the dielectric elastomer membrane as well as an inner padding layer (26), and the padding layer (26) is filled with an incompressible liquid and has at least one outlet valve (28) which is closed in a normal state and open in an emergency state.

2. The blood circulation assistance device (22) as claimed in claim 1, **characterized in that** the thickness of the padding layer (26) corresponds at least to one contraction stroke of the contraction layer (24).

3. The blood circulation assistance device (22) as claimed in claim 1, **characterized in that** the contraction layer (24) consists of a number of annular sections which are separately electrically controllable in a time-staggered sequence.

4. The blood circulation assistance device (22) as claimed in claim 3, **characterized in that** from 3 to 250 annular sections of the contraction layer (24) are provided.

5. The blood circulation assistance device (22) as claimed in claim 3, **characterized in that** the padding layer (26) consists of the same number of liquid-filled annular chambers, which are respectively located below the annular sections of the contraction layer, wherein each annular chamber has its own outlet valve.

6. The blood circulation assistance device (22) as claimed in one of the preceding claims, **characterized in that** the padding layer (26) communicates via the outlet valve (28) with a collecting bag or an outlet line for passage out of the body.

7. The blood circulation assistance device (22) as claimed in one of the preceding claims, **characterized in that** the incompressible liquid of the padding layer (26) is physiologically acceptable and can be released into the body of the living being via the outlet valve (28).

8. The blood circulation assistance device (22) as claimed in claim 6, **characterized in that** the incompressible liquid of the padding layer (26) comprises at least one substance with positive inotropic activity.

9. The blood circulation assistance device (22) as claimed in one of the preceding claims, **characterized in that** the control device comprises an emergency state detection unit which determines a state of emergency if the power supply for the contraction layer (24) falls below a minimum value for a specified period of time.

10. A blood circulation assistance system comprising a blood circulation assistance device (22) as claimed in one or more of the preceding claims, a control device (30), a power supply device and at least one sensor device for detecting the cardiac cycle.

## Revendications

1. Dispositif d'assistance à la circulation sanguine (22) pour un cœur d'un être vivant, comprenant une manchette pour la sollicitation par pression périodique du cœur au moyen d'au moins une membrane diélectrique en élastomère qui est contrôlable par impulsions par un dispositif de commande en synchronisation avec un battement de cœur pour transporter le sang, la manchette étant conçue pour être emboîtée à l'extérieur au-dessus du cœur (10) et présentant pour ce faire une forme intérieure qui est adaptée au contour extérieur du cœur du moins dans la zone située à l'extérieur des ventricules du cœur, **caractérisé en ce que** la manchette est composée d'une couche de contraction extérieure (24) entourée de la membrane diélectrique en élastomère ainsi que d'une couche intérieure formant coussin (26) et que la couche formant coussin (26) est remplie d'un liquide incompressible et présente au moins une soupape de sortie (28) qui est fermée en état normal et ouverte en état d'urgence.

2. Dispositif d'assistance à la circulation sanguine (22) selon la revendication 1, **caractérisé en ce que** la couche formant coussin (26) présente une épaisseur qui correspond au moins à une course de contraction de la couche de contraction (24).

3. Dispositif d'assistance à la circulation sanguine (22) selon la revendication 1, **caractérisé en ce que** la couche de contraction (24) est composée d'un certain nombre de sections annulaires qui sont contrôlables électriquement séparément et séquentiellement de manière décalée dans le temps.

4. Dispositif d'assistance à la circulation sanguine (22) selon la revendication 3, **caractérisé en ce que** 3 à 250 sections annulaires de la couche de contraction (24) sont prévues.

5. Dispositif d'assistance à la circulation sanguine (22) selon la revendication 3, **caractérisé en ce que** la couche formant coussin (26) est composée du même nombre d'espaces annulaires remplis de fluide qui se trouvent respectivement en dessous des sections annulaires de la couche de contraction, chaque espace annulaire présentant sa propre soupape de sortie.

6. Dispositif d'assistance à la circulation sanguine (22) selon une des revendications précédentes, **caractérisé en ce que** la couche formant coussin (26) communique par la soupape de sortie (28) avec une poche de récupération ou une conduite de sortie pour évacuation hors du corps.

7. Dispositif d'assistance à la circulation sanguine (22) selon une des revendications précédentes, **caractérisé en ce que** le liquide incompressible de la couche formant coussin (26) est compatible physiologiquement et peut être diffusé dans le corps de l'être vivant par la soupape de sortie (28).

8. Dispositif d'assistance à la circulation sanguine (22) selon la revendication 6, **caractérisé en ce que** le liquide incompressible de la couche formant coussin (26) comprend au moins une substance à effet positif inotrope.

9. Dispositif d'assistance à la circulation sanguine (22) selon une des revendications précédentes, **caractérisé en ce que** le dispositif de commande comprend une unité de constatation d'état d'urgence qui constate un état d'urgence lorsque l'alimentation en énergie pour la couche de contraction (24) dépasse une valeur minimum pendant une durée prédéfinie.

10. Système d'assistance à la circulation sanguine comprenant un dispositif d'assistance à la circulation sanguine (22) selon une ou plusieurs des revendications précédentes, un dispositif de contrôle (30), un dispositif d'alimentation énergétique et au moins un dispositif de détection pour la détection du cycle cardiaque.
